# EUROPEAN PATENT APPLICATION

(11) **EP 4 830 889 A1**
(43) Date of publication of application: **28.10.2026**
(21) Application number: 26171592.4
(22) Date of filing: 10.04.2026
(51) Int. Cl.: B26D 5/00, G16H 20/13, B65B 5/10, B65B 61/06

(54) **MACHINE FOR THE AUTOMATED SINGULARIZATION OF PHARMACEUTICAL PRODUCTS, AND CORRESPONDING SINGULARIZATION METHOD**

(30) Priority: 10.04.2025 IT 202500007908
(71) Applicant: Vaccari Mauro S.r.l., 40062 Molinella (BO) (IT)
(72) Inventor: Vaccari, Nicola, 40062 Molinella (BO) (IT)
(74) Representative: Petraz, Gilberto Luigi

(57) **Abstract**

Machine (10) and method for the automated singularization of pharmaceutical products (50) packaged in corresponding sealed cavities (110) of tablet packs (100), or blisters, comprising at least one detection and cutting station (13) configured to receive one or more tablet packs (100) at a time and to obtain a corresponding plurality of single sealed cavities (110), each containing a corresponding one of said pharmaceutical products (50), wherein the detection and cutting station (13) is provided with at least one detection device (24) to carry out at least one optometric detection of each of said tablet packs (100), and with a cutting device (25) comprising at least one laser emitting member (28) configured to cut each of said tablet packs (100), separating said single sealed cavities (110) according to specific cutting lines (T).

## Description

### FIELD OF THE INVENTION

The present invention concerns a machine for the automated singularization of pharmaceutical products, such as tablets or pills for example, of the type stored and distributed in tablet packs or blisters.

### BACKGROUND OF THE INVENTION

It is known that some types of pharmaceutical products, such as for example tablets, pills or suchlike, hereinafter more simply drugs, are packaged in blisters, in which the drugs are individually housed in corresponding sealed cavities, the latter distributed according to a desired and specific positioning matrix, as a function of the number and size of the drugs, as provided.

It is also known that, particularly for some types of active pharmaceutical ingredients, or for some particular therapies, a patient is provided with a predefined number of drugs, limited to the fulfillment of the pharmacological treatment procedures. In other words, the diagnosed pharmaceutical dosage may be chosen, on each occasion, by the physician, depending on the actual biomedical and/or care conditions of the patient.

However, from an industrial point of view, the drugs are still packaged in blisters that contain a number of drugs, regardless of what the individual pharmaceutical dosage choices provided by the doctors will be. Therefore, for each diagnosis, medical personnel, or pharmacists, may find themselves with the need to singularize the drugs, still keeping them in the corresponding sealed cavities, so as to supply them to the patient in the expected number.

These drug singularization operations can be carried out either manually, for example by cutting the blister with scissors, or with semi-automatic equipment, for example by die-cutting.

However, especially because of the need to always have traceability of drugs for their storage and supply, both manual and also semi-automatic die-cutting require using an entire blister, even to supply a limited number of drugs, with the risk of wasting the remaining unused drugs.

In a practical example, in which a patient is given two drugs a day for five days, if starting from a blister containing a number of eighteen or twenty drugs, almost half of the blister is wasted, because it remains unused, since it at least partly loses the quality and dosage indications of the packaged drugs.

It is clear that this known procedure produces a high level of drug waste, which results in a pharmaceutical and economic waste. In this sense, it suffices to consider that some drugs, for example those used for the treatment of rare and oncological diseases, have extremely high unit costs, for example of the order of hundreds of thousands of euros per year per patient, and that hospital facilities invest a total of several billions of euros per year to buy these drugs, with waste caused by singularization estimated at about 5-7%.

In addition to the problems of waste, known systems do not always guarantee a correct and repeatable singularization of the drugs, in particular with blisters of different formats and packaging conformations, since both the manual cutting and also the die cutting can intervene too close to the drug, possibly compromising the seal of the cavity that contains it. This disadvantage, which can occur both due to human distraction and also due to geometric and/or positioning incompatibility of the bister with respect to the die used, can lead to a deterioration of the drug, generating further waste or risks for the patient.

With known solutions, both the waste resulting from the singularization and also the one caused by cutting imperfections entail management costs for the disposal of compromised or expired drugs, with considerable repercussions from an environmental impact point of view.

To attempt to overcome, at least in part, the above disadvantages affecting the solutions known in the art, FR 2810974 discloses a system for the automatic preparation of drug doses comprising a plurality of different operating stations. In this system, it is provided to associate the blisters on a film equipped with perforations that define a reference system, thanks to which the system knows the relative position of the drug tablets with respect to the perforations.

Subsequently, the blisters are cut so as to singularize the doses of drugs by means of a robot equipped with mechanical cutting means comprising two half-jaws provided with blades that cooperate to make a shear cut. The system provides the preventive storage, for each drug to be distributed, of the blister sizes and of the position and sizes of the alveoli, each containing one respective tablet, as well as of the possible cutting lines to separate each unit dose individually. This information can be input manually by an operator, or optically detected by means of a camera or scanner and a shape recognition system.

However, this known solution has some disadvantages. First of all, the system operates on the basis of pre-stored geometric data for each type of drug, thus assuming prior knowledge of the blister format and not allowing automatic adaptation to blisters of variable or non-standard geometries. Secondly, mechanical scissor cutting is inadequate for processing blisters made of transparent, semi-transparent or multilayer plastic materials, typical of many modern pharmaceutical packages, with the risk of compromising the seal of the cavities containing the drugs. In addition, the system requires a necessary preliminary step of associating the blisters with the film, resulting in operational complexity and the need for dedicated tooling for each blister format.

There is therefore the need to perfect an automated singularization machine and develop a method that can overcome at least one of the disadvantages of the state of the art.

To achieve this, it is necessary to solve the technical problem of preventing any waste of pharmaceutical products resulting from their singularization.

In particular, one purpose of the present invention is to provide a machine and develop a method for singularizing pharmaceutical products that allow to maintain the traceability of each singularized drug.

Another purpose of the present invention is to provide a machine and develop a method that are highly versatile and guarantee a correct and repeatable singularization of the drugs, even with blisters of different formats and packaging conformations.

A further purpose of the present invention is to provide a machine and develop a method that allow to reduce the environmental impact linked to managing the disposal of spoiled or expired drugs.

Another purpose of the present invention is to provide a machine and develop a method capable of singularizing pharmaceutical products through a cutting operation suitable to cut the material that the blisters are made of, by means of clear cutting lines in intended positions, without the risk of compromising the seal of the cavities containing the pharmaceutical products.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes and to solve the technical problem described above in a new and original way, also achieving considerable advantages compared to the state of the prior art, a machine according to the present invention for the automated singularization of pharmaceutical products, such as tablets, pills and suchlike, for example, packaged in corresponding sealed cavities of tablet packs, or blisters, substantially comprises at least one detection and cutting station, which is configured both to receive one or more tablet packs at a time and also to obtain a corresponding plurality of single sealed cavities, each containing a corresponding pharmaceutical product.

In the solution according to the present invention, the detection and cutting station is provided with at least one detection device, which is configured to carry out at least one optometric detection of each tablet pack to obtain an image that reflects the real geometry of each of the tablet packs, and with a cutting device comprising at least one laser emitting member, which is configured to cut each of the tablet packs, thus separating the single sealed cavities, according to specific cutting lines obtained dynamically as a function of the image that reflects the real geometry obtained from the detection device.

The term "dynamically" indicates that the cutting lines are determined in a dynamic manner for each package, on the basis of the image that reflects the real geometry of the package that has been detected by the detection device, and are not standard or previously stored.

In doing so, being able to configure specific cutting lines on each occasion as a function of the real sizes and conformations of the tablet packs, the cutting of the individual sealed cavities, that is, the singularization of the pharmaceutical products, occurs in an optimized manner, thus preventing any waste of drugs.

The singularization machine constructed according to the present invention thus guarantees a correct and repeatable singularization of drugs, even with blisters of different formats and packaging conformations, thus reducing accidental drug waste.

An additional advantage of the present invention is that the machine thus constructed allows to reduce the environmental impact of managing the disposal of spoiled, unusable, or expired drugs, thus reducing overall management costs.

These advantages of the solution according to the present invention will appear even clearer when compared with what provided in the prior art, especially, but not only, with regard to drugs used to treat rare and oncological diseases, where a reduction in waste caused by drug singularization can lead to significant economic savings, even of several percentage points compared to the annual pharmaceutical expenditure of a healthcare facility which, considering the overall annual amounts invested by such facilities, can be in the order of hundreds of thousands, if not several million, euros per year.

In accordance with another aspect of the present invention, the machine comprises a processing unit, which is electronically connected at least to the detection device and to the cutting device.

In this way, the processing unit can receive the data relating to the image that reflects the real geometry of the blister, obtained from the detection device. The data received can then be processed as a function of set comparison parameters, so as to dynamically calculate the corresponding cutting lines. At this point, the processing unit, since it is also electronically connected to the cutting device, commands the latter to cut each of the tablet packs along the cutting lines.

Moreover, the singularization machine according to the present invention can comprise at least one storage station disposed operationally upstream of the detection and cutting station and configured to house a plurality of tablet packs, grouped into homogeneous groups according to characteristics of size and shape.

Always according to the present invention, the singularization machine can comprise a packaging and labelling station disposed downstream of the detection and cutting station, configured both to house a specific number of individual sealed cavities in a container, and also to print the identification data of the corresponding pharmaceutical product on a corresponding label to be applied on the container.

In this way, the singularization machine according to the present invention allows to maintain the traceability of each singularized drug, thus reducing functional waste and lowering any costs caused by waste.

In fact, by identifying the pharmaceutical products contained in each container with the label, these can be administered, even at a later time, to other patients, or sent to other hospitals or healthcare facilities, depending on requirements, thus optimizing the use of the pharmaceutical products.

Advantageously, the processing unit can be electronically connected at least to the storage station and to the packaging and labeling station, so that it is possible to receive data relating to the characteristics of size and shape of the tablet packs, and thus command the labeling device in relation to the printing to be performed on the corresponding label, reporting the identifying data of the corresponding pharmaceutical product.

In accordance with some advantageous embodiments of the present invention, the detection device comprises at least one optical scanning member, which is configured to sequentially perform a specific number of optical scans of the corresponding tablet pack, so as to reconstruct, through optical triangulation, at least one three-dimensional vector image of the scanned tablet pack. The vector reconstruction allows to process the cutting lines with extreme precision, allowing them to be modified on each occasion, even with the same type of tablet pack, independently of the alignment of the latter, of its conformation, or of the number of individual sealed cavities provided.

In this embodiment, the processing unit is configured to compare the three-dimensional vector image with a sample image of an equivalent tablet pack, in order to verify that they correspond in size and conformation and, as a function of the actual spatial position of the tablet pack and the sealed cavities, outline the cutting lines.

In accordance with other advantageous embodiments of the present invention, wherein the tablet pack is a multi-layer structure comprising at least one layer made of transparent or semi-transparent plastic materials, the laser emitting member is configured to emit a specific UV laser beam suitable to cut the tablet pack along the cutting lines. In this way, the cut is very clean, precise and thin, as well as effective even in tablet packs made of the aforementioned materials, typically used for the production of pharmaceutical blisters.

Advantageously, the machine according to the present invention can also comprise a manipulator member, which is operationally interposed between the detection device and the cutting device, so that each tablet pack can be disposed first in a detection position with reference to the scanning member, and then in a cutting position with reference to the laser emitting member. In this way, the tablet pack is moved between the two scanning and cutting members, respectively, with corresponding alignments, essentially defining a univocal piece zero for each individual tablet pack manipulated, on each occasion, by the manipulator member.

According to another aspect of the present invention, there is provided a method for the automated singularization of pharmaceutical products packaged in corresponding sealed cavities of tablet packs, or blisters, comprising at least one detection and cutting step in which, by means of at least one detection and cutting station, from one or more of the tablet packs at a time, a corresponding plurality of single sealed cavities is obtained, each one containing a corresponding one of the pharmaceutical products. The detection and cutting step comprises at least one detection sub-step in which at least one optometric detection of each of the tablet packs is carried out by means of at least one detection device, in order to obtain an image that reflects the real geometry of each of the tablet packs, and a cutting sub-step in which each of the tablet packs is cut by means of a cutting device comprising at least one laser emitting member, separating the single sealed cavities according to the cutting lines calculated dynamically as a function of the image that reflects the real geometry obtained from the detection device.

According to one aspect of the present invention, the method comprises at least one processing step in which, by means of a processing unit electronically connected at least to the detection device and to the cutting device, the image that reflects the real geometry is received from the detection device and processed as a function of set comparison parameters, and the corresponding cutting lines are calculated dynamically, on the basis of which the cutting device is commanded to cut each of the tablet packs according to the cutting lines.

According to one aspect of the present invention, the detection sub-step comprises the sequential execution of a certain number of optical scans of each of the tablet packs, and the reconstruction by optical triangulation of at least one three-dimensional vector image of the tablet pack.

According to one aspect of the present invention, the cutting sub-step is carried out by means of a laser emitting member configured to emit a UV laser beam suitable to cut transparent, semi-transparent and multi-layered plastic materials.

### DESCRIPTION OF THE DRAWINGS

These and other aspects, characteristics and advantages of the present invention will become apparent from the following description of an embodiment, given as a non-restrictive example with reference to the attached drawings wherein:
- fig. 1 schematically shows a layout of a machine for the automated singularization of pharmaceutical products according to the present invention;
- fig. 2 shows a schematic three-dimensional view of a blister containing pharmaceutical products to be singularized with the machine of fig. 1;
- fig. 3 is a schematic and three-dimensional view of an embodiment of the machine of fig. 1;
- fig. 4 is a three-dimensional view of an enlarged detail of a storage station of the machine of fig. 3;
- fig. 5 shows a sectioned detail of fig. 4;
- fig. 6 shows an enlarged detail of a detection and cutting station of the machine of fig. 3;
- fig. 7 shows a sectioned detail of fig. 6;
- fig. 8 shows an enlarged detail of a packaging and labeling station of the machine of fig. 3; and
- fig. 9 shows a sectioned detail of fig. 8.

We must clarify that the phraseology and terminology used in the present description, as well as the figures in the attached drawings also in relation as to how described, have the sole function of better illustrating and explaining the present invention, their purpose being to provide a non-limiting example of the invention itself, since the scope of protection is defined by the claims.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF AN EMBODIMENT OF THE PRESENT INVENTION

With reference to the attached drawings, there is described a machine 10 according to the present invention for the automated singularization of pharmaceutical products, such as for example tablets 50, or pills, of the type stored and distributed in tablet packs, or blisters 100.

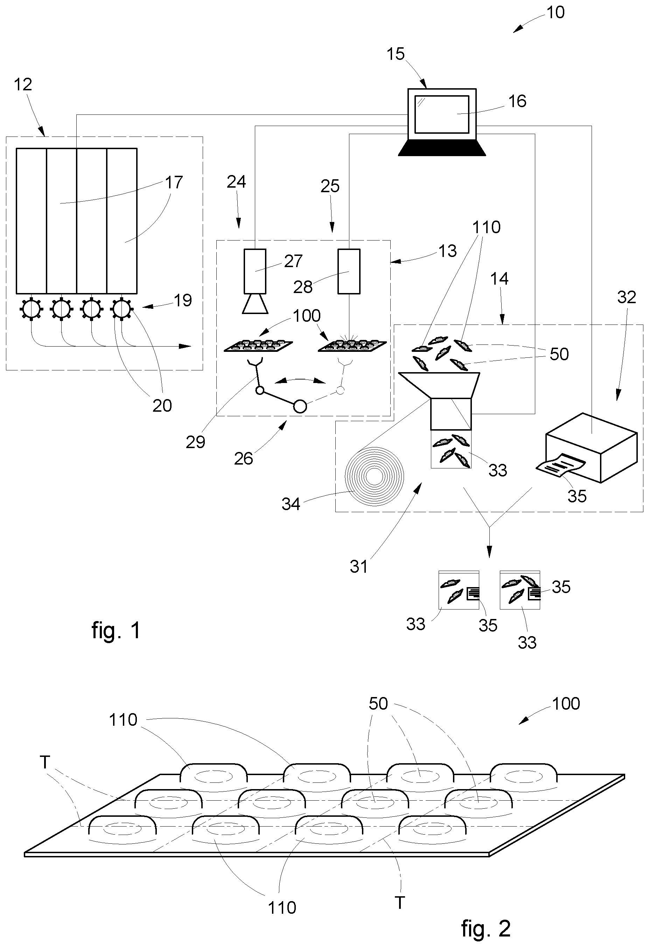

In particular, the blister 100 (fig. 2) is normally configured to define a plurality of sealed cavities 110, each containing a single tablet 50 and distributed according to a specific distribution matrix. Depending on the sizes, or the type of tablet 50 packaged, or the box format or other, the matrix and the sizes of the blister 100 can be different from each other, even considerably.

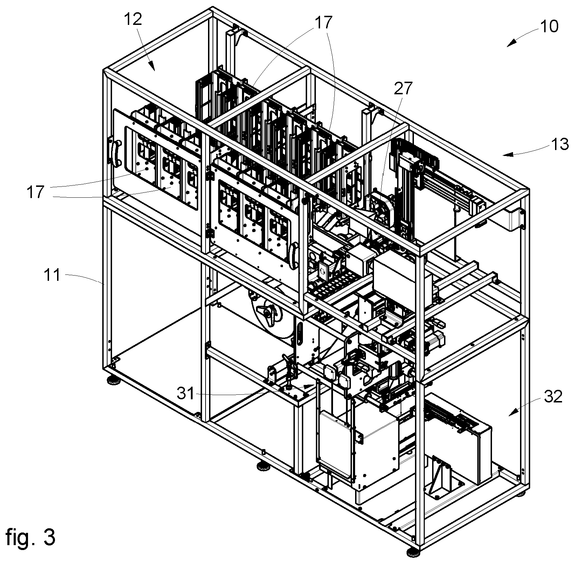

The machine 10 substantially comprises a main structure 11 (fig. 3) inside which, as schematically shown in fig. 1, a storage station 12 of the blisters 100, a detection and cutting station 13 and a packaging and labelling station 14 are provided in operating sequence. Also part of the machine 10 is a processing unit 15 electronically connected to the aforementioned stations 12, 13 and 14, so as to command and reciprocally coordinate their operation. The processing unit 15 also comprises an interface section 16 disposed at least partly outside the main structure 11 and configured to allow a user to program the processing unit 15 itself, in order to select and request a desired amount of specific tablets 50, program the management of new types of blisters 100, or other functions that may become necessary for the correct and effective management of the machine 10.

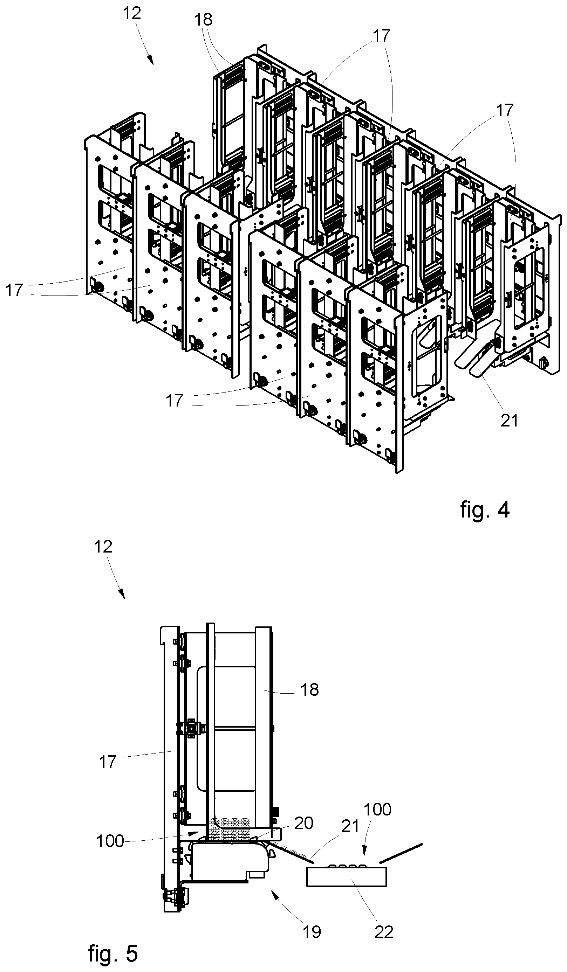

The storage station 12 (figs. 4 and 5) substantially comprises a plurality of modular containers 17, for example about a dozen, each of which is configured to house a certain number of blisters 100 stacked vertically on each other, all containing the same type of tablet 50 and having the same characteristics of size and shape.

In this way, each modular container 17 contains the same type of blister 100 that can be identified according to desired identification parameters. For example, for each modular container 17, it can be provided to computer load the data relating to the active ingredient or the expiry of the drug, or the number of tablets 50 provided and other, into the processing unit 15.

Each modular container 17 has, externally, univocal sizes and mounting inserts, while it has internal movable side walls 18 which allow the internal sizes of the modular container 17 to be adapted to the actual sizes of the type of blister 100 for which it is intended. In this way, the stacked storage of different sizing types of blister 100 can be provided in a same modular container 17. Although not specifically shown, each modular container 17 can provide quick mounting inserts, of a substantially known type, for mounting to the main structure 11, in order to facilitate the operations of tooling and changing set-up of the storage station 12.

Each modular container 17 is combined at the lower part with an automated extractor 19, which is embodied as a carousel pusher 20, which cooperates selectively with the lowermost blister 100 of the stack contained in the modular container 17, in order to push it transversely toward an extraction chute 21. A conveyor belt 22 is disposed in cooperation with the modular containers 17, so that each extraction chute 21 conveys the extracted blister 100 toward the conveyor belt 22. The latter is movable so as to convey the extracted blisters 100 toward the detection and cutting station 13.

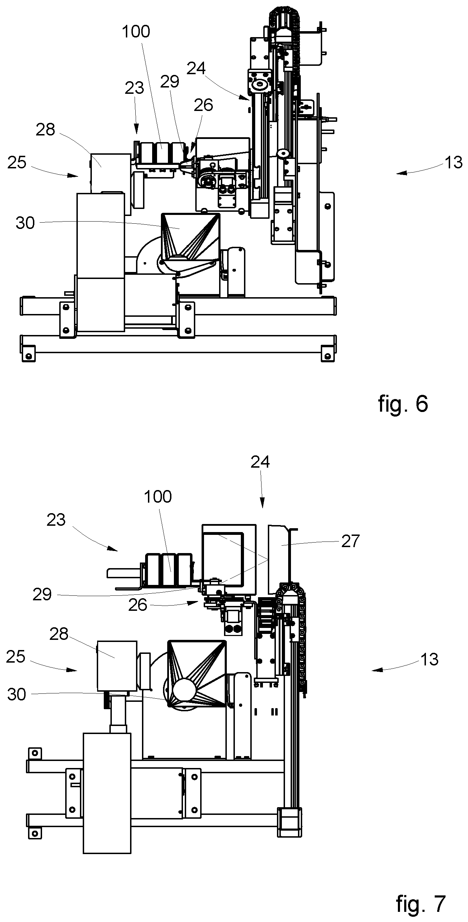

The detection and cutting station 13 (figs. 6 and 7) is configured to receive one or more blisters 100 at a time and to obtain a corresponding plurality of individual sealed cavities 110, each containing a corresponding tablet 50.

In particular, the detection and cutting station 13 comprises a detection device 24 which is configured to carry out at least one optometric detection of each blister 100, and a cutting device 25, the latter configured to cut each blister 100 so as to separate the individual sealed cavities 110 according to specific cutting lines T, which are obtained as a function of the optometric detection carried out by the detection device 24.

Unlike what is schematized in fig. 1, in the embodiment shown in figs. 3, 6 and 7, the detection device 24 and the cutting device 25, for operational convenience, operate with the blister 100 kept in a substantially vertical position. However, other advantageous solutions are not excluded, in which the blister 100 is in a substantially horizontal, inclined, or horizontal position in one station, and vertical in the other, or other positions, as well as in motion, depending on the technology chosen for the detection device 24 and for the cutting device 25.

A manipulator member 26 operationally interposed between the detection device 24 and the cutting device 25 is also part of the detection and cutting station 13, precisely in order to manipulate each blister 100 between the detection device 24 and the cutting device 25.

The detection and cutting station 13 further comprises an edging member 23, which is operationally disposed immediately downstream of the conveyor belt 22, and is suitable to edge, that is, align on a same side, the blisters 100 fed by the conveyor belt 22. In this way, a "piece zero" is substantially defined on the blister 10, thus being able to adapt subsequent operations to the actual sizes and conformations of the individual and different blisters 100 being fed.

The detection device 24 comprises at least one optical scanning member 27 configured to sequentially perform a specific number of optical scans, even more than ten, of the corresponding blister 100, so as to allow to reconstruct, by optical triangulation, at least one three-dimensional vector image of the scanned blister 100. This vector graphic processing can be carried out directly by the scanning member 27 and subsequently sent to the processing unit 15, that is, only the images are sent, and the processing itself is performed by the processing unit 15.

In any case, the three-dimensional vector image obtained is compared by the processing unit 15 with a sample image of an equivalent blister 100, initially so as to verify their correspondence in size and conformation and, subsequently, as a function of the actual spatial position of the blister 100 and of the sealed cavities 110, and any other parameters, in order to outline the suitable cutting lines T specific for the blister 100.

In other embodiments, included within the scope of the present invention, the detection device 24 can reconstruct the image of the blister 100 even using other techniques, for example different from the aforementioned optical triangulation.

The cutting device 25 comprises a laser emitting member 28, movable in space, preferably on at least two axes, so as to emit a specific UV laser beam and cut the blister 100 by following the cutting lines T. In particular, the cutting device 25 is electronically connected to the processing unit 15, so as to receive from the latter data relating to the cutting lines T along which to direct its UV laser beam.

The manipulator member 26 substantially comprises a holding gripper 29, movable by means of a movement kinematics 30, so as to pick up the blisters 100 aligned by the edging member 23 and position them first in a detection position, in which the blister 100 is in a univocally determined position with respect to the optical scanning member 27, and subsequently in a cutting position, in which the blister 100 is in a position, different from the previous one, univocally determined with respect to the laser emitting member 28.

In this way, each blister 100, after being aligned, is picked up by the holding gripper 29 and brought into position, in this specific case substantially vertical, in front of the scanning member 27, for the acquisition of the necessary images. The same holding gripper 29 translates, in this specific case into a substantially vertical position, the same blister 100 and positions it in cooperation with the laser emitting member 28, so that the sealed cavities 110 can be deliberately separated along the corresponding cutting lines T, processed by the processing unit 15. These sealed cavities 110 are fed, in a desired number, to a hopper 30 of the packaging and labelling station 14.

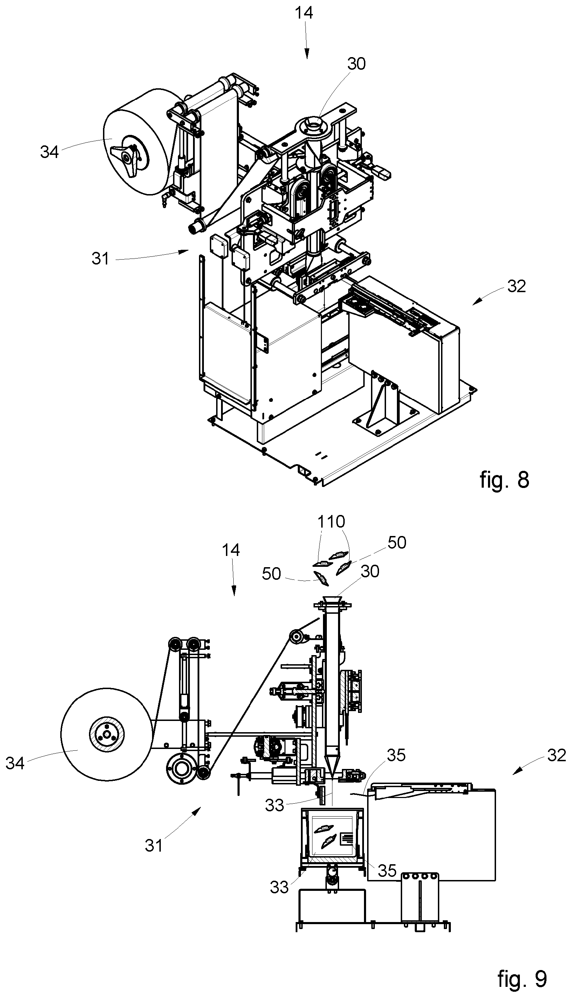

The packaging and labelling station 14 (figs. 8 and 9) comprises, in addition to the hopper 30, a packaging device 31 and a labelling device 32.

The packaging device 31 is of a substantially known type and is able to define, by hot welding starting from a coil 34, a container, or plastic pouch 33, around the sealed cavities 110 separated and fed in a desired number through the hopper 30.

The labelling device 32 is configured to print on a corresponding label 35 the identification data of the tablets 50 contained in the individual sealed cavities 110 disposed in the pouch 33. The same labelling device 32 is configured to apply the printed label 35 on the pouch 33.

In particular, both the packaging device 31 and also the labelling device 32 are electronically connected to the processing unit 15, so that the latter commands to the former the number of sealed cavities 110, and therefore of tablets 50, to be packaged for each pouch 33, and to the latter the identification data of the tablets 50, as detected by the starting modular containers 17.

The machine 10 according to the present invention thus provides, at the end of the method according to the present invention, a pouch 33 labelled with the label 35, inside which there are a desired number of tablets 50. The labelling of each pouch 33 allows, even in the future, to trace the tablets 50 contained therein, so that they can be made available for future uses, or be supplied to other facilities on request.

It is clear that modifications and/or additions of parts may be made to the machine 10 and to the method as described heretofore, without thereby departing from the field and scope of the present invention, as defined by the claims.

It is also clear that, although the present invention has been described with reference to some specific examples, a person of skill in the art will be able to achieve other equivalent forms of a machine for the automated singularization of pharmaceutical products, and corresponding singularization method, having the characteristics as set forth in the claims and hence all coming within the field of protection defined thereby.

In the following claims, the sole purpose of the references in brackets is to facilitate their reading and they must not be considered as restrictive factors with regard to the field of protection defined by the claims.

## Claims

1. Machine (10) for the automated singularization of pharmaceutical products (50) packaged in corresponding sealed cavities (110) of tablet packs (100), or blisters, **characterized in that** it comprises at least one detection and cutting station (13) configured to receive one or more of said tablet packs (100) at a time and to obtain a plurality of single sealed cavities (110), wherein the detection and cutting station (13) is provided with at least one detection device (24) to carry out at least one optometric detection of each of said tablet packs (100) so as to obtain an image that reflects the real geometry of each of said tablet packs (100), and with a cutting device (25) comprising at least one laser emitting member (28) configured to cut each of said tablet packs (100), thus separating said single sealed cavities (110), each containing a corresponding one of said pharmaceutical products (50), according to specific cutting lines (T) obtained dynamically as a function of said image that reflects the real geometry obtained from said detection device (24).

2. Machine (10) as in claim 1, **characterized in that** it comprises a processing unit (15) electronically connected at least to said detection device (24) and to said cutting device (25), configured at least to receive said image that reflects the real geometry from said detection device (24), to process said image as a function of set comparison parameters, to dynamically calculate said corresponding cutting lines (T) and to command said cutting device (25) to cut each of said tablet packs (100) along said cutting lines (T).

3. Machine (10) as in claim 1 or 2, **characterized in that** it comprises at least one storage station (12) disposed operationally upstream of said detection and cutting station (13) and having a plurality of modular containers (17), each of which is configured to house a number of said tablet packs (100) with specific characteristics of size and shape.

4. Machine (10) as in one or other of the previous claims, **characterized in that** it comprises at least one packaging and labelling station (14) disposed operationally downstream of said detection and cutting station (13) and having at least one packaging device (31) configured to house a specific number of said individual sealed cavities (110) in a container (33), and at least one labelling device (32) configured to print the identification data of the corresponding pharmaceutical product (50) on a corresponding label (35) and apply the latter on said container (33).

5. Machine (10) as in claims 2, 3 and 4, **characterized in that** said processing unit (15) is electronically connected at least to said storage station (12) and to said packaging and labelling station (14), and is configured at least to receive said specific characteristics of size and shape of said tablet packs (100) so as to command said labelling device (32) to print said corresponding label (35) with said identification data of the corresponding pharmaceutical product (50).

6. Machine (10) as in one or other of the previous claims, **characterized in that** said detection device (24) comprises at least one optical scanning member (27) configured to sequentially perform a specific number of optical scans of the corresponding one of said tablet packs (100) and to reconstruct, through optical triangulation, at least one three-dimensional vector image of said corresponding one of said tablet packs (100).

7. Machine (10) as in claim 6, **characterized in that** said processing unit (15) is configured to compare said three-dimensional vector image with a sample image of an equivalent tablet pack (100) in order to verify their correspondence in size and conformation and, as a function of the actual spatial position of the tablet pack (100) and of the sealed cavities (110), outline said cutting lines (T).

8. Machine (10) as in one or other of the previous claims, wherein said tablet pack (100) is a multi-layer structure comprising at least one layer made of transparent, semi-transparent plastic materials, **characterized in that** said laser emitting member (28) is configured to emit a specific UV laser beam suitable to cut said tablet pack (100).

9. Machine (10) as in one or other of the previous claims, **characterized in that** it comprises a manipulator member (26) operationally interposed between said detection device (24) and said cutting device (25), and configured to dispose each of said tablet packs (100) first in a detection position with reference to said scanning member (27), and then in a cutting position with reference to said laser emitting member (28).

10. Machine (10) as in one or other of the previous claims, **characterized in that** said detection and cutting station (13) comprises an edging member (23) configured to align said tablet packs (100) on a same side, defining a piece zero on each tablet pack (100).

11. Method for the automated singularization of pharmaceutical products (50) packaged in corresponding sealed cavities (110) of tablet packs (100), or blisters, **characterized in that** it comprises at least one detection and cutting step in which, by means of at least one detection and cutting station (13), from one or more of said tablet packs (100) at a time, a corresponding plurality of single sealed cavities (110) is obtained, each one containing a corresponding one of said pharmaceutical products (50), said detection and cutting step comprising at least one detection sub-step in which at least one optometric detection of each of said tablet packs (100) is carried out by means of at least one detection device (24) in order to obtain an image that reflects the real geometry of each of said tablet packs (100), and a cutting sub-step in which each of said tablet packs (100) is cut by means of a cutting device (25) comprising at least one laser emitting member (28), separating said single sealed cavities (110) according to the cutting lines (T) calculated dynamically as a function of said image that reflects the real geometry obtained from said detection device (24).

12. Method as in claim 11, **characterized in that** it comprises at least one processing step in which, by means of a processing unit (15) electronically connected at least to said detection device (24) and to said cutting device (25), said image that reflects the real geometry is both received from said detection device (24) and also processed as a function of set comparison parameters, and said corresponding cutting lines (T) are calculated, and said cutting device (25) is commanded to cut each of said tablet packs (100) according to said cutting lines (T).

13. Method as in claim 11 or 12, **characterized in that** said detection sub-step comprises the sequential execution of a certain number of optical scans of each of said tablet packs (100) and the reconstruction by optical triangulation of at least one three-dimensional vector image of said tablet pack (100).

14. Method as in one or other of claims from 11 to 13, **characterized in that** said cutting sub-step is carried out by means of a laser emitting member (28) configured to emit a UV laser beam suitable to cut transparent, semi-transparent and multi-layered plastic materials.

15. Method as in claims 13 and 14, **characterized in that** it comprises a manipulation sub-step in which, by means of a manipulator member (26), each of said tablet packs (100) is first disposed in a detection position first with reference to a scanning member (27), and subsequently in a cutting position with reference to said laser emitting member (28).
